(19)

Europäisches
Patentamt

European
Patent Office

Office européen
des brevets

(11) **EP 1 604 187 B1**

(12) **EUROPEAN PATENT SPECIFICATION**

(45) Date of publication and mention
of the grant of the patent:
**14.05.2008 Bulletin 2008/20**

(21) Application number: **04720433.4**

(22) Date of filing: **12.03.2004**

(51) Int Cl.:
***G01N 21/35*** (2006.01)  ***E21B 49/10*** (2006.01)

(86) International application number:
**PCT/US2004/007612**

(87) International publication number:
**WO 2004/083833 (30.09.2004 Gazette 2004/40)**

(54) **A METHOD AND APPARATUS FOR DOWNHOLE QUANTIFICATION OF METHANE USING NEAR INFRARED SPECTROSCOPY**

VERFAHREN UND VORRICHTUNG ZUR BOHRLOCH-QUANTIFIZIERUNG DES METHANS MITTELS NAHINFRAROT-SPEKTROSKOPIE

PROCEDE ET DISPOSITIF POUR L'EVALUATION QUANTITATIVE DE METHANE EN TROU DE FORAGE, PAR SPECTROSCOPIE DANS L'INFRAROUGE PROCHE

(84) Designated Contracting States:
**GB NL**

(30) Priority: **14.03.2003 US 454897 P**

(43) Date of publication of application:
**14.12.2005 Bulletin 2005/50**

(73) Proprietor: **Baker Hughes Incorporated
Houston, TX 77019-2118 (US)**

(72) Inventor: **DIFOGGIO, Rocco
Houston, TX 77099 (US)**

(74) Representative: **Vigars, Christopher Ian
HASELTINE LAKE,
Redcliff Quay,
120 Redcliff Street
Bristol BS1 6HU (GB)**

(56) References cited:
**GB-A- 2 334 097         US-A- 4 994 671
US-A- 5 892 586         US-A- 5 939 717
US-B1- 6 274 865**

• **DONG C. ET AL: "In-Situ Contamination Monitoring and GOR Measurement of Formation Fluid Samples" PROCEEDINGS SPE ASIA PACIFIC OIL AND GAS CONFERENCE AND EXHIBITION, 8 October 2002 (2002-10-08), pages 635-643, XP008080064**

**Description**

**Field of the Invention**

The invention relates generally to a method and apparatus for quantifying the weight percentage of methane or the gas oil ratio for a crude oil sample downhole by using the sample's absorbance at two specially selected optical channels. The center wavelengths and bandwidths of these channels were selected by a complex simulation.

**Background of the Invention**

In wellbore exploration, typically drilling mud such as oil-based mud and synthetic-based mud are used. Filtrates from these muds generally invade the hydrocarbon bearing formation through the borehole wall. Thus, samples taken from the formation contain drilling mud filtrate contamination. Thus, a sufficient volume of fluid must pumped from the formation to reduce the filtrate contamination in the sample to an acceptable level. Open-hole sampling is an effective way to acquire representative formation fluid samples. Formation fluid sample acquisition and analysis allows determination of critical information for assessing the economic value of reserves. In addition, optimal production strategies can be designed to handle these complex fluids. In openhole sampling, initially, the flow from the formation contains a considerable quantity of filtrate, but as filtrate is drained from the formation, the flow increasingly becomes richer in formation fluid and less filtrate appears in the flow. That is, the composition of fluid flowing from the formation progresses towards a higher percentage of native formation fluid but a lower percentage of filtrate as pumping continues and the filtrate that had invaded the formation is depleted.

Thus, fluid being pumped from a wellbore undergoes a clean-up process in which the purity of the sample increases over time as filtrate is gradually removed from the formation so that less filtrate appears in the sample. As the composition of the sampled formation fluid changes, so do the optical and physical properties of the sampled fluid, such as optical absorption, fluorescence, refractive index, density, and viscosity. A number of different measurements are used to determine various optical and physical properties of a fluid downhole in real time. Measuring these properties of the fluid therefore provides insight into a sample's purity.

When extracting fluids from a formation, it is desirable to quantify the cleanup progress, that is, the degree contamination from filtrate in the formation fluid sample in real time. If it is known that there is too much filtrate contamination in the sample (e.g., more than about 10% filtrate), then there is little reason to collect a formation fluid sample in a sample tank. One should wait until the contamination level drops to an acceptable level. On the other hand, if by pumping for a relatively long time, it is possible to achieve an only slightly better filtrate contamination level, an operator may end up wasting very expensive rig time and also risks the very costly possibility of allowing a tool to become stuck in the wellbore.

When pumping first begins, the fluid being pumped contains a large amount of mud filtrate contamination but the fluid filtrate percentage is decreasing at the fastest rate. This process of decreasing fluid filtrate contamination is referred to as sample clean up. Later, the pumped fluid contains less contamination but the fluid filtrate percentage decreases at a slower rate. One way to monitor cleanup is to monitor the increase in gas oil ratio (GOR) as pumping continues and the flow from the formation cleans up from mostly gas-free oil-based mud filtrate and to mostly gas-containing oil.

Oil companies are also very interested in knowing the GOR of the crude oils that they find downhole independent of using GOR as a cleanup monitor. Thus, there is a need for a method and apparatus for determining GOR in real time downhole.

U.S. Patent 4,994,671 to Safinya teaches a device for analyzing the composition of formation fluids, and more particularly to apparatus and methods for using near infrared spectral analysis to determine the quantities of gas, water and various types of oils In a formation fluid. The Safinya device determines the composition of the fluid sample by comparing the spectrum of the emitted light and the spectrum of the detected light which has been affected by the fluid sample.

U.S. Patent 5,892,586 to Thony teaches a gas detection process that includes the steps of emitting a first radiation at an absorption wavelength for the gas to be detected with a first switched microlaser and producing a first signal (S) representative of the radiation quantity scattered by the gas in response to the interaction between the molecules or atoms of the gas and the first radiation. The process is described as used for detecting gases in the atmosphere.

The article by Dong C. et al: "In-Situ Contamination Monitoring and GOR Measurement of Formation Fluid Samples" PROCEEDINGS SPE ASIA PACIFIC OIL AND GAS CONFERENCE AND EXHIBITION, 8 October 2002, pages 635-643, discloses monitoring in real time the relative variation of the methane content of a formation fluid, by comparing the optical density at an absorption wavelength of methane with a background optical density.

U.S. Patent 5,939,717 to Mullins teaches the detection of methane density in a fluid by integrating the amplitude of the absorption spectrum in a given waveband.

Summary of the Invention

This method and apparatus of the present invention utilizes spectroscopy to estimate the weight fraction of methane and the corresponding Gas Oil Ratio (GOR) for a methane-in-crude-oil mixture. A method and apparatus are provided to determine the gas oil ratio from the weight fraction of methane, which is determined spectroscopically. The present invention provides a method and apparatus for optical analysis of formation fluids using near infrared (NIR) illumination, which provides a measurement of optical absorbance at wavelengths of 1670 nanometers and 1682 nanometers. The methods of this invention correlate the absorption at these two wavelengths to the weight percent methane and GOR. A borehole apparatus for measuring the spectral absorbance of formation fluids includes a testing region, a conduit for directing formation fluid into the testing region, a light source emitting at least near infrared rays into the testing region, a spectral detector optically coupled to the testing region, and a processor coupled to the spectral detector. The testing region is an optically transparent cell or chamber which is located between the light source and the spectral detector such that light directed from the light source to the spectral detector is passes through formation fluid. The spectral detector is in one example is a filter spectrograph, which measures the spectrum of the light which has been transmitted through the formation fluid in the testing region.

The present invention provides a method and apparatus for quantifying methane and GOR downhole using a complex simulation and regression selection process to obtain specially selected optical filters having particular selected center wavelengths and bandpasses (11nm full width half maximum FWHM) to quantify the weight percentage of methane or the GOR for a crude oil sample in real time downhole. Specifically, the invention is a method of determining weight percent methane and GOR for formation fluid samples being pumped from a formation surrounding a wellbore by a wireline tool or a monitoring-while-drilling formation tester to obtain weight percent methane and to estimate GOR for a formation fluid sample.

Unlike Mullins US 6,476,384 (Mullins '384), which describes a method for determining GOR based on two wavelengths, the first located near a methane-gas spectral peak and the second located near a liquid-hydrocarbon spectral peak (representing oil), the present invention uses two wavelengths that are both near a single spectral peak for methane (i.e., two regions of the same methane peak). Also, unlike Mullins '384, which based its spectral GOR determination equations on a training set of binary mixtures of n-heptane (representing oil) and methane, the present invention bases its spectral GOR equations on synthetic mixtures of methane and dead crude oils. A dead crude oil is one for which little or no gas remains in the crude oil because it was not stored under pressure and therefore the gas in it was released. Unlike heptane, which is visibly clear, real crude oils have considerable amounts of dark-colored asphaltenes. The tails of the optical absorption peaks of asphaltenes usually produce the equivalent of a baseline offset and some baseline tilt in the long-wavelength region (1620-1780 nm) that includes both the methane and liquid hydrocarbon peaks. Also, the liquid hydrocarbon peak is more complicated (has features associated with aromatics, saturates, etc.) for the case of a mixture of hundreds of hydrocarbons (dead crude oils) than for the case of a single pure solvent (the saturate, n-heptane). For both reasons, in contrast to Mullins '384, the present invention uses stock tank crude oils rather than n-heptane to represent downhole crude oil in the modeling for GOR or weight percent methane.

Brief Description Of The Figures

Other objects and advantages of the invention will become apparent upon reading the following detailed description and upon reference to the accompanying drawings in which:

**Figure 1** is a schematic diagram of a exemplary embodiment of the present invention deployed on a wireline in a downhole environment;

**Figure 2** is a schematic diagram of an exemplary embodiment of the present invention deployed on a drill string in a monitoring while drilling environment;

**Figure 3** is a schematic diagram of a exemplary embodiment of the present invention deployed on a flexible tubing in a downhole environment;

**Figure 4** is a schematic diagram of an exemplary embodiment of the present invention as deployed in a wireline downhole environment showing a cross section of a wireline formation tester tool;

**Fig. 5** is a diagram of the Fluid Characterization Module;

**Fig. 6** is illustration of a regression analysis over two wavelengths and temperature for weight percent methane and GOR; and

**Fig. 7** illustrates three spectra of methane at various temperatures and pressures and one representative crude oil spectrum.

**Fig. 8** illustrates a flow chart of functions performed by the present invention.

**Detailed Description Of An Exemplary Embodiment**

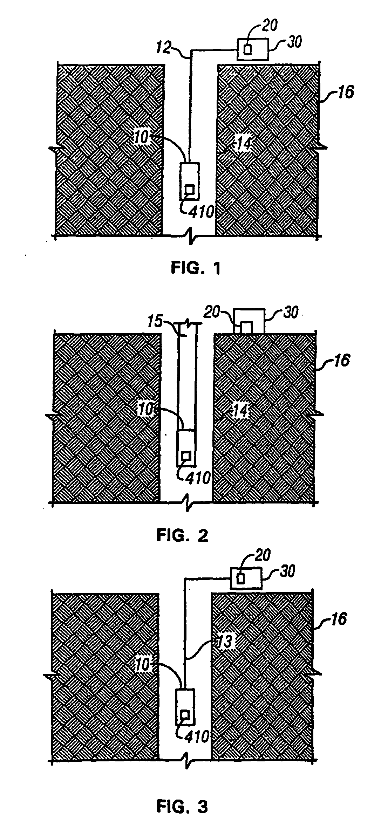

**Fig. 1** is a schematic diagram of an exemplary embodiment of the present invention deployed on a wireline in a downhole environment. As shown in **Fig. 1**, a downhole tool **10** containing a optical analyzer **410** of the present invention is deployed in a borehole **14**. The borehole is formed in formation **16**. Tool **10** is deployed via a wireline **12**. Data from the tool **10** is communicated to the surface to a computer processor **20** with memory inside of an intelligent completion system **30**. **Fig. 2** is a schematic diagram of a exemplary embodiment of the present invention deployed on a drill string **15** in a monitoring while drilling environment. **Fig. 3** is a schematic diagram of an exemplary embodiment of the present invention deployed on a flexible tubing **13** in a downhole environment.

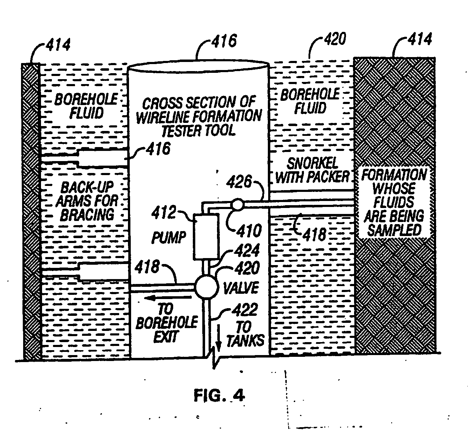

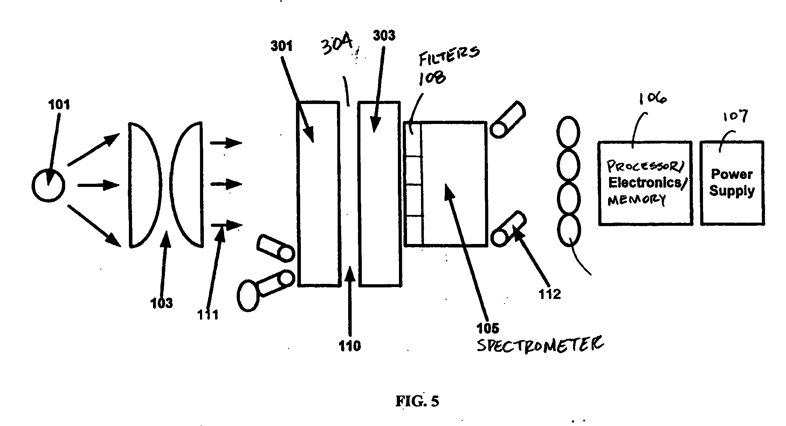

**Figure 4** is a schematic diagram of an exemplary embodiment of the present invention as deployed from a wireline downhole environment showing a cross section of a wireline formation tester tool. As shown in **Figure 4,** the tool **416** is deployed in a borehole **420** filled with borehole fluid. The tool **416** is positioned in the borehole by backup arms **416**. A packer with a snorkel **418** contacts the borehole wall for extracting formation fluid from the formation **414**. Wellbore fluid can be drawn from the wellbore also by not extending the snorkel to the wall and pumping fluid from the wellbore instead of the formation. Tool **416** contains optical analyzer **410**, shown in **Fig. 5**, disposed in flow line **426**. The optical analyzer response is monitored to determine weight percent methane and GOR of the formation fluid. Pump **412** pumps formation fluid from formation **414** into flow line **426**. Formation fluid travels through flow line **424** into valve **420**, which directs the formation fluid to line **422** to save the fluid in sample tanks or to line **418** where the formation fluid exits to the borehole.

**Fig. 5** illustrates a schematic representation for a downhole fluid characterization module, as, for example, the Baker Atlas SampleView^SM tool. A light source **101** (e.g. tungsten light bulb) emits light toward a formation or wellbore sample **110**. Light from light source **101** is collimated by a collimating lens device **103** lying between the light source and the sample **110**. The collimated light **111** is incident generally perpendicular to a first sapphire window **301** adjacent sample **110**. Sapphire windows **301** and **303** lie generally perpendicular to the collimated beam of light and are separated by a gap or channel **304** enabling a fluid sample **110** to flow between them. The flow channel **304** can be flow line **426**. Reflected and fluoresced light can be monitored over time in the spectrometer **105** and processor/electronics/memory **106** comprising a central processing unit, control circuitry and memory (not shown) to determine sample properties such as weight percent methane and GOR. The exemplary tool shown in **Fig. 5** is fitted with ultraviolet, near infrared, mid-infrared (UV/NIR/MIR) wavelength light sources **112**, which can be turned on when the tungsten light source **101** is turned off. The same spectrometer, comprising single wavelength filters **108** over spectrometer photodiodes, enables collecting the crude oil spectra for light transmitted, reflected or fluoresced in the UV, NIR, MIR bands associated the sample in flow channel **304.**

The present invention provides a multiple channel spectrometer, in the current example comprising 24 channels of visible, near infrared (NIR) and mid infrared (MIR) light, which are shown through the sample **110** and filtered out into separate wavelength bands.

Two filters center wavelengths are carefully selected to be at 1670 nm and 1682 nm and to have bandpasses of 11nm full width half maximum (FWHM). These two wavelengths were selected based on a complex simulation in which spectra of methane at various pressures and temperatures were added in random amounts to the spectra of 10 randomly chosen crude oil spectra from a data base of 500 spectra of diverse crude oil samples from around the world. The laboratory spectra were degraded to 11 nm resolution to approximate what is currently the best resolution of commercially available long-wavelength high temperature optical filters that are suitable for downhole use. Various step-forward and step-backward regressions with substitution were performed on the simulated mixtures over a wavelength region of 1500-1900 nm to determine the best-correlating center wavelengths, which turned out to be 1670 nm and 1682 nm, and their corresponding correlation equations. Temperature and pressure, which are non-optical parameters, were also used in the regressions along with the selected wavelengths to obtain the equation for weight fraction of methane.

According to the invention, a borehole apparatus for measuring the spectral peaks of a methane region includes a testing region, a conduit for directing formation fluid into the testing region, a light source emitting at least near infrared rays into the testing region, a spectral detector optically coupled to the testing region, and a processor coupled to the spectral detector. The testing region is a transparent cell or chamber, which is located between the light source and the spectral detector such that light directed from the light source to the spectral detector is interrupted by formation fluid. The spectral detector is preferably a spectrometer, which measures the spectrum of the light, which has been transmitted through the formation fluid in the testing region.

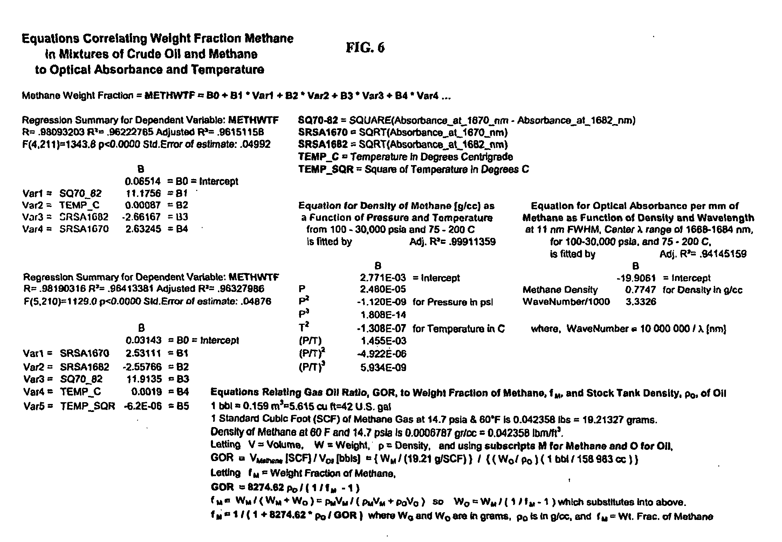

As shown in **Fig. 6**, the optimal center wavelengths 1670 nm and 1682 nm were derived from a regression analysis on a much broader wavelength region of 1500nm-1900nm. **Fig. 6** illustrates the equations for calculation of methane weight and GOR from the selected channel measurements. **Fig. 6** also shows empirical correlations developed for the weight fraction of methane in mixtures of methane and crude oil in the current example of the invention. The correlation equation gives the weight fraction of methane as a function of the mixture absorbance at two wavelengths (1670 nm and 1682 nm) and temperature. **Fig. 6** also illustrates empirical correlations associated with the present invention and developed for the density of methane as a function of pressure and temperature and for the optical absorption per millimeter of methane as a function of methane density and wavenumber (a wavenumber is 10,000,000 / wavelength expressed in nanometers) regardless of pressure and temperature.

As shown in **Fig. 6**, the equation for correlating weight fraction methane in mixtures of crude oil and methane to optical absorbance and temperature are illustrated.

The form of the equation for methane weight fraction in the present invention is that of an offset constant, B0, plus a first constant, B1, times a first variable, Var1, plus a second constant, B2, times a second variable, Var2, and so on to an N-th constant and variable.

$$\text{METHWTF} = \text{Methane Weight Fraction} = \text{B0} + \text{B1} * \text{Var1} + \text{B2} * \text{Var2} + \text{B3} * \text{Var3} + \text{B4} * \text{Var4} \ldots \text{BN} * \text{VarN}$$

The following is a first example of a Regression Summary for Dependent Variable: METHWTF
R= .98093203 $R^2$= .96222765 Adjusted $R^2$= .96151158
$F_{(4,211)}$=1343.8 p<0.0000 Std.Error of estimate: .04992

|  |  | B |  |  |
|---|---|---|---|---|
|  |  | 0.065139686 | = B0 | = Intercept |
| Var1 = | SQ70_82 | 11.17561047 | = B1 |  |
| Var2 = | TEMP_C | 0.000869088 | = B2 |  |
| Var3 = | SRSA1682 | -2.661667658 | = B3 |  |
| Var4 = | SRSA1670 | 2.63244987 | = B4 |  |

Where:

SQ70-82 = SQUARE(Absorbance_at_1670_nm - Absorbance_at_1682_nm)

SRSA1670 = SQRT(Absorbance_at_1670_nm)

SRSA1682 = SQRT(Absorbance_at_1682_nm)

TEMP_C = Temperature in Degrees Centrigrade

TEMP_SQR = Square of Temperature in Degrees C

The following is a second example of a Regression Summary for Dependent Variable: METHWTF
R= .98190316 $R^2$= .96413381 Adjusted $R^2$= .96327986
$F_{(5,210)}$=1129.0 p<0.0000 Std.Error of estimate: .04876

|  |  | B |  |  |
|---|---|---|---|---|
|  |  | 0.031427753 | = B0 | = Intercept |
| Var1 = | SRSA1670 | 2.531111433 | = B1 |  |
| Var2 = | SRSA1682 | -2.557658783 | = B2 |  |
| Var3 = | SQ70_82 | 11.91350402 | = B3 |  |
| Var4 = | TEMP_C | 0.0019 | = B4 |  |
| Var5 = | TEMP_SQR | -6.2E-06 | =B5 |  |

Baseline offset refers to a simultaneous and equal increase in the absorbance of whatever optical channels are being monitored. In this example, it would refer to an increase in the absorbance at both 1670 nm and 1682 nm by the same amount. Inspection of the first and second example equations shows that these equations have little sensitivity to baseline offset. This fact is one of the benefits of basing one's model on asphaltene-containing crude oils rather than on clear solvents. Doing so provides insensitivity to the presence of asphaltenes, which, to first order, simply appears as a baseline offset over the narrow wavelength region of 1670 nm to 1682 nm. The degree of this baseline offset depends upon the type of asphaltenes and upon their concentration.

The dominant term of the first equation is the square of the difference (the slope) between 1670 nm and 1682 nm. A slope is completely invariant to baseline offset. Also, taken together, the third and fourth terms are approximately equal to the slope between 1670 nm and 1682 nm on a plot of the square root of absorbance versus wavelength and so have low sensitivity to baseline offset. In like manner, the second equation is very insensitive to baseline offset and thus insensitive to the presence of asphaltenes, which inevitably are found in any real crude oil.

We developed the following empirical equation (Adjusted $R^2$ = .99911359) for the density of methane [g/cc] as a function of pressure and temperature from 100 - 30,000 psia and 75 - 200° C.

|   | B |   |
|---|---|---|
|   | 2.771 E-03 | = Intercept |
| P | 2.480E-05 |   |
| $P^2$ | -1.120E-09 | for Pressure in psi |
| $P^3$ | 1.808E-14 |   |
| $T^2$ | -1.308E-07 | for Temperature in C |
| (P/T) | 1.455E-03 |   |
| $(P/T)^2$ | -4.922E-06 |   |
| $(P/T)^3$ | 5.934E-09 |   |

We also developed the following empirical equation (Adjusted $R^2$ = .94145159) for optical absorbance per millimeter of methane as function of density and wavelength over 1668-1684 nm, 100-30,000 psia, and 75 - 200 C, assuming a bandpass of 11 nm FWHM.

|   | B |   |
|---|---|---|
|   | -19.9061 | = Intercept |
| Methane Density | 0.7747 | for Density in g/cc |
| WaveNumber/1000 | 3.3326 |   |

where, Wavenumber = 10,000,000 / $\lambda$, [nm]

The following definitions and equations let us relate GOR to Weight Fraction of Methane, $f_M$, and Stock Tank Oil Density, $\rho_O$.

1 bb1 = 0.159 $m^3$ = 5.615 cu ft = 42 U.S. gal

1 Standard Cubic Foot (SCF) of Methane Gas at the standard conditions of 14.7 psia and 60°F is 0.042358 lbs =19.21327 grams.

So, the density of Methane at 60° F and 14.7 psia is 0.0006787 gr/cc = 0.042358 $lbm/ft^3$.

The Gas Oil Ratio is defined as

$$GOR = V_{Methane} [SCF] / V_{Oil} [bbls]$$

so

$$GOR = \{ W_M / (19.21 \text{ g/SCF}) \} / \{ ( W_O / \rho_O )( 1 \text{ bbl} / 158\,983 \text{ cc} ) \}$$

Let $f_M$ = Weight Fraction of Methane, and let the subscripts V = Volume, W = Weight, p = Density, M = Methane, and O = Oil. Then:

$$GOR = 8274.62 \, \rho_O / ( 1 / f_M - 1 )$$

$$f_M = W_M / (W_M + W_O) = \rho_M V_M / (\rho_M V_M + \rho_O V_O) \text{ or } W_O = W_M / (1 / f_M - 1).$$

Rearranging the equation for GOR we obtain:

$$f_M = 1 / (1 + 8274.62 * \rho_O / GOR)$$

where $W_G$ and $W_O$ are in grams, $\rho_O$ is in g/cc, and $f_M$ = Wt. Frac. of Methane

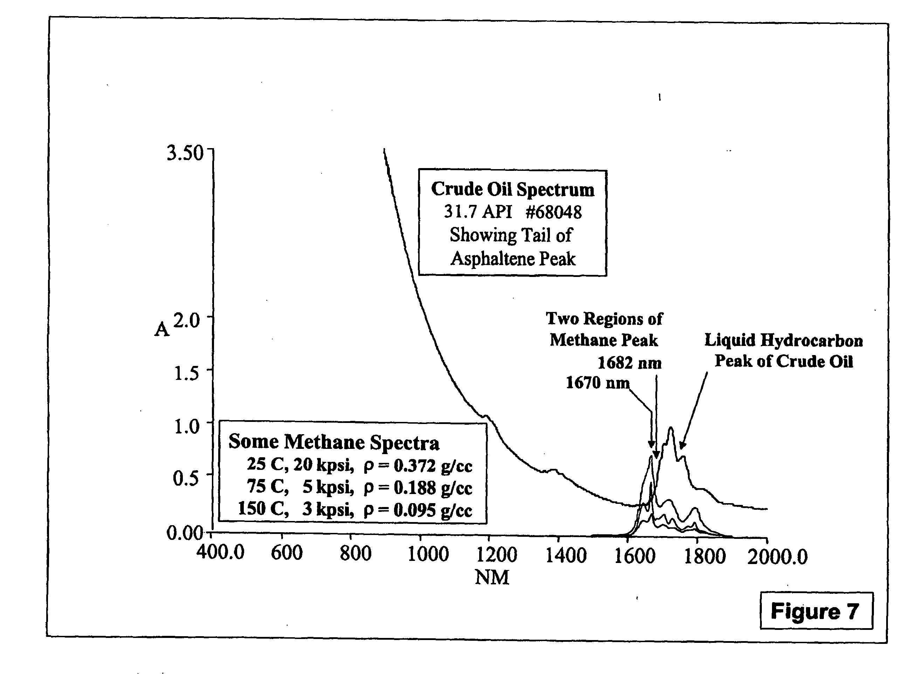

Figure 7 shows three spectra of methane at various pressures and temperatures and the positions of the 1670 nm and 1682 nm channels relative to the methane peak. The higher the mass density [g/cc] of the methane the taller the methane peak. Note that the 1670 nm channel is almost at the pinnacle of the methane peak whereas the 1682 nm channel is slightly to the right of the pinnacle on the right shoulder of the methane peak.

Also shown is a representative crude oil spectrum. The rising left edge of this spectrum is the asphaltene peak of the crude oil. The liquid hydrocarbon peak of the crude oil is near 1740 nm. In a mixture of methane and crude oil, the methane peak will appear to sit on top of the right-hand tail of the asphaltene peak. That is why it is important that the weight-percent-methane model be insensitive to baseline offset.

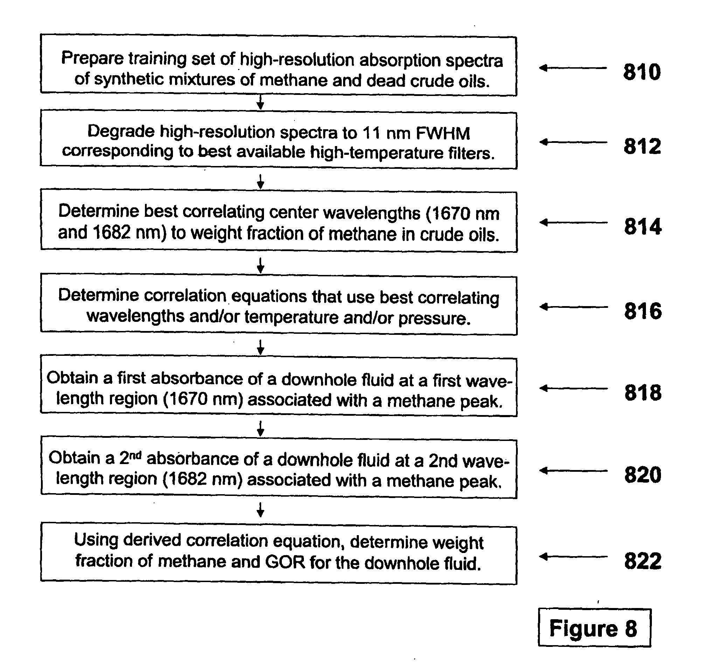

Turning now to **Fig. 8,** a diagram of some of the functions performed in the tool and associated processor functions in present example of the invention is illustrated. In block **810** in the present example of the invention a training set of high-resolution absorption spectra of synthetic mixtures of methane and dead crude oils is prepared. In block **812** in the present example of the invention these high-resolution spectra are degraded to 11 nm FWHM resolution corresponding to best available high-temperature filters.. In block **814** in the present example of the invention the best correlating center wavelengths (1670 nm and 1682 nm) to weight fraction of methane in crude oils are determined. In block **816** in the present example of the invention the correlation equations that use these best correlating wavelengths and/or temperature and/or pressure are determined. In block **818** in the present example of the invention a first absorbance of a downhole fluid at a first wave-length region (1670 nm) associated with a methane peak is obtained. In block **820** in the present example of the invention a second absorbance of a downhole fluid at a second wavelength region (1682 nm) associated with a methane peak is obtained. In block **822** in the present example of the invention a weight fraction of methane and corresponding GOR for the downhole fluid are determined using the earlier derived correlation equation and the sample cleanup can be also monitored based on a change in weight percent methane or GOR.

The present invention has been described as method and apparatus operating in a downhole environment in the preferred embodiment, however, the present invention may also be embodied as a set of instructions on a computer readable medium, comprising ROM, RAM, CD ROM, Flash or any other computer readable medium, now known or unknown that when executed cause a computer to implement the method of the present invention. While a preferred embodiment of the invention has been shown by the above invention, it is for purposes of example only and not intended to limit the scope of the invention, which is defined by the following claims.

**Claims**

1.   A method for quantifying the weight percent methane in real-time in a wellbore environment, comprising obtaining the fluid downhole; measuring a first optical absorbance value for the fluid at a first wavelength region associated with a methane peak and **characterized by**

     measuring a second optical absorbance value for the fluid at a second wavelength region associated with the methane peak: and
     determining weight percent methane for the fluid sample from the first and second measured optical absorbance values using a correlation equation which gives the weight percent of methane utilising the first and second optical absorbance values, and a temperature value for the fluid.

2.   The method of claim1, further **characterized in that** the first wavelength region has a center wavelength of 1670 nanometers; and the second wavelength has a center wavelength of 1682 nanometers.

3.   The method of claim 1, further **characterized by**:

     by correlating pressure.

4. The method of claim 1 further **characterized by**:

   determining a gas oil ratio for the sample based on the weight percent methane.

5. The method of claim 1, further **characterized by**:

   monitoring sample cleanup based on a change in weight percent methane.

6. The method of claim 1, further **characterized by**:

   correlating based on synthetic mixtures of methane and dead crude oils.

7. The method of claim 1, further **characterized by**:

   filtering an optical absorbance measurement with a 11 nm full width half maximum filter.

8. The method of claim 1, further **characterized in that** the first wavelength region has a center wavelength of 1670 nanometers and the second wavelength has a center wavelength of 1682 nanometers;
   correlating weight percent methane, pressure and temperature with optical absorbance at the first and second wavelength regions; and
   determining a gas oil ratio based on the weight percent methane,

9. An apparatus for quantifying the weight percent methane in real-time in a wellbore environment, comprising:

   a tool (10) for obtaining a fluid downhole;
   a spectrometer (105) for measuring a first optical absorbance value for the fluid at a first wavelength region associated with a methane peak and measuring a second optical absorbance value for the fluid at a second wavelength region associated with the methane peak; and **characterized by**:

   a processor function adapted to determine weight percent methane for the fluid sample from the first and second measured optical absorbance values using a correlation equation which gives the weight percent of methane utilising the first and second measured optical absorbance values, and a temperature value for the fluid.

10. The apparatus of claim 9, further **characterized in that** the first wavelength region has a center wavelength of 1670 nanometers; and the second wavelength has a center wavelength of 1682 nanometers.

11. The apparatus of claim 9, the processor function further **characterized by** a function for correlating pressure.

12. The apparatus of claim 9 further **characterized by**:

    a processor function for determining a gas oil ratio for the sample based on the weight percent methane.

13. The apparatus of claim 9. further **characterized by**:

    a processor function for monitoring sample cleanup based on a change in weight percent methane.

14. The apparatus of claim 9, the processor function further **characterized by** a function for correlating based on synthetic mixtures of methane and dead crude oils.

15. The apparatus of claim 9, further **characterized by**:

    a filter for filtering an optical absorbance measurement with a 11 nm full width half maximum filter.

16. The apparatus of claim 9, further **characterized in that** the first wavelength region has a center wavelength of 1670 nanometers and the second wavelength has a center wavelength of 1682 nanometers, the processor function further **characterized by** a
    function for correlating weight percent methane, pressure and temperature with optical absorbance at the first and second wavelength regions and a function for determining a gas oil ratio based on the weight percent methane.

17. A computer readable medium containing executable instructions that when executed by a computer perform a method for quantifying the weight percent methane in real-time in a wellbore environment, comprising:

obtaining a fluid downhole;
measuring a first optical absorbance value for the fluid at a first wavelength region associated with a methane peak; and **characterized by**:

measuring a second optical absorbance value for the fluid at a second wavelength region associated with the methane peak; and
determining weight percent methane for the fluid sample from the first and second measured optical absorbance values using a correlation equation which gives the weight percent of methane utilising the first and second optical absorbance values, and a temperature value for the fluid.

18. The medium of claim 17, further **characterized in that** the first wavelength region has a center wavelength of 1870 nanometers; and the second wavelength has a center wavelength of 1682 nanometers.

19. The medium of claim 17, further **characterized by**:

correlating pressure.

20. The medium of claim 17 further **characterized by**:

determining a gas oil ratio for the sample based on the weight percent methane.

21. The medium of claim 17, further **characterized by**:

monitoring sample cleanup based on a change in weight percent methane.

22. The medium of claim 17, further **characterized by**:

correlating based on synthetic mixtures of methane and dead crude oils.

23. The medium of claim 17, further **characterized by**:

filtering an optical absorbance measurement with a 11 nm full width half maximum filter.

24. The medium of claim 17, further **characterized in that** the first wavelength region has a center wavelength of 1670 nanometers and the second wavelength has a center wavelength of 1682 nanometers; correlating weight percent methane, pressure and temperature with optical absorbance at the first and second wavelength regions; and determining a gas oil ratio based on the weight percent methane.

**Patentansprüche**

1. Verfahren zum mengenmäßigen Bestimmen von Methan in Gewichtsprozent in Realzeit in einer Bohrlochumgebung, bei welchem das Fluid aus dem Bohrloch erhalten wird und ein erster optischer Absorptionsgradwert für das Fluid bei einem ersten Wellenlängenbereich gemessen wird, der einer Methanspitze zugeordnet ist, **dadurch gekennzeichnet, dass** ein zweiter optischer Absorptionsgradwert für das Fluid bei einem zweiten Wellenlängenbereich gemessen wird, der der Methanspitze zugeordnet ist, und dass das Methan in Gewichtsprozent für die Fluidprobe aus dem ersten und zweiten gemessenen optischen Absorptionsgradwert unter Verwendung einer Korrelationsgleichung bestimmt wird, die das Methan in Gewichtsprozent unter Verwendung des ersten und zweiten optischen Absorptionsgradwerts und einen Temperaturwert für das Fluid liefert.

2. Verfahren nach Anspruch 1, welches weiterhin **dadurch gekennzeichnet ist, dass** der erste Wellenlängenbereich eine zentrale Wellenlänge von 1670 nm und der zweite Wellenlängenbereich eine zentrale Wellenlänge von 1682 nm hat.

3. Verfahren nach Anspruch 1, welches weiterhin durch einen korrelierenden Druck **gekennzeichnet** ist.

4. Verfahren nach Anspruch 1, welches weiterhin durch Bestimmen eines Gas-Öl-Verhältnisses für die Probe basierend auf dem Methan in Gewichtsprozent **gekennzeichnet** ist.

5. Verfahren nach Anspruch 1, welches weiterhin durch Überwachen der Probenentgasung basierend auf einer Gewichtsprozentänderung des Methans **gekennzeichnet** ist.

6. Verfahren nach Anspruch 1, welches weiterhin durch Korrelieren basierend auf synthetischen Mischungen von Methan und entgasten Rohölen **gekennzeichnet** ist.

7. Verfahren nach Anspruch 1, welches weiterhin **dadurch gekennzeichnet ist, dass** eine Messung des optischen Absorptionsgrads mit einem 11nm-Vollbreiten-Halbmaximumsfilter filtriert wird.

8. Verfahren nach Anspruch 1, welches weiterhin **dadurch gekennzeichnet ist, dass** der erste Wellenlängenbereich eine zentrale Wellenlänge von 1670 nm und der zweite Wellenlängenbereich eine zentrale Wellenlänge von 1682 nm hat, dass das Methan in Gewichtsprozent, der Druck und die Temperatur mit dem optischen Absorptionsgrad bei dem ersten und zweiten Wellenlängenbereich korreliert werden und dass ein Gas-Öl-Verhältnis basierend auf dem Methan in Gewichtsprozent bestimmt wird.

9. Vorrichtung zum mengenmäßigen Bestimmen von Methan in Gewichtsprozent in Realzeit in einer Bohrlochumgebung,

> - mit einem Gerät (10) zum Erhalten eines Fluids aus dem Bohrloch
> - mit einem Spektrometer (105) zum Messen eines ersten optischen Absorptionsgradwerts für das Fluid bei einem ersten Wellenlängenbereich, der einer Methanspitze zugeordnet ist, und zum Messen eines zweiten optischen Absorptionsgradwerts für das Fluid bei einem zweiten Wellenlängenbereich, der der Methanspitze zugeordnet ist,

**gekennzeichnet durch**

> - eine Prozessorfunktion, die zum Bestimmen von Methan in Gewichtsprozent für die Fluidprobe aus dem ersten und zweiten gemessenen optischen Absorptionsgradwert unter Verwendung einer Korrelationsgleichung angepasst ist, die das Methan in Gewichtsprozent unter Verwendung des ersten und zweiten gemessenen optischen Absorptionsgradwerts und einen Temperaturwert für das Fluid angibt.

10. Vorrichtung nach Anspruch 9, welche weiterhin **dadurch gekennzeichnet ist, dass** der erste Wellenlängenbereich eine zentrale Wellenlänge von 1670 nm und der zweite Wellenlängenbereich eine zentrale Wellenlänge von 1682 nm hat.

11. Vorrichtung nach Anspruch 9, bei welcher die Prozessorfunktion weiterhin durch eine Funktion für einen korrelierenden Druck **gekennzeichnet** ist.

12. Vorrichtung nach Anspruch 9, welche weiterhin durch eine Prozessorfunktion zum Bestimmen eines Gas-Öl-Verhältnisses für die Probe basierend auf dem Methan in Gewichtsprozent **gekennzeichnet** ist.

13. Vorrichtung nach Anspruch 9, welche weiterhin durch eine Prozessorfunktion zum Überwachen der Probenentgasung basierend auf einer Gewichtsprozentänderung des Methans **gekennzeichnet** ist.

14. Vorrichtung nach Anspruch 9, bei welcher die Prozessorfunktion weiterhin durch eine Funktion zum Korrelieren basierend auf synthetischen Mischungen von Methan und entgasten Rohölen **gekennzeichnet** ist.

15. Vorrichtung nach Anspruch 9, welche weiterhin durch einen Filter zum Filtern einer optischen Absorptionsgradmessung mit einem 11nm-Vollbreiten-Halbmaximumsfilter **gekennzeichnet** ist.

16. Vorrichtung nach Anspruch 9, welche weiterhin **dadurch gekennzeichnet ist, dass** der erste Wellenlängenbereich eine zentrale Wellenlänge von 1670 nm und der zweite Wellenlängenbereich eine zentrale Wellenlänge von 1682 nm hat und die Prozessorfunktion weiterhin durch eine Funktion zum Korrelieren des Methans in Gewichtsprozent, des Drucks und der Temperatur mit einem optischen Absorptionsgrad bei dem ersten und zweiten Wellenlängenbereich und durch eine Funktion zum Bestimmen eines Gas-Öl-Verhältnisses basierend auf dem Methan in Gewichtsprozent **gekennzeichnet** ist.

17. Rechnerlesbares Medium, das ausführbare Instruktionen enthält, die, wenn sie von einem Rechner ausgeführt werden, ein Verfahren zum mengenmäßigen Bestimmen von Methan in Gewichtsprozent in Realzeit in einer Bohrlochumgebung ausführen, bei welchem

   - ein Fluid aus dem Bohrloch erhalten wird und
   - ein erster optischer Absorptionsgradwert für das Fluid bei einem ersten Wellenlängenbereich gemessen wird, der einer Methanspitze zugeordnet ist, **gekennzeichnet durch**
   - Messen eines zweiten optischen Absorptionsgradwerts für das Fluid bei einem zweiten Wellenlängenbereich, der der Methanspitze zugeordnet ist, und
   - Bestimmen von Methan in Gewichtsprozent für die Fluidprobe aus dem ersten und zweiten gemessenen optischen Absorptionsgradwert unter Verwendung einer Korrelationsgleichung, welche das Methan in Gewichtsprozent unter Verwendung des ersten und zweiten optischen Absorptionsgradwerts und einen Temperaturwert für das Fluid liefert.

18. Medium nach Anspruch 17, welches weiterhin **dadurch gekennzeichnet ist, dass** der erste Wellenlängenbereich eine zentrale Wellenlänge von 1670 nm und der zweite Wellenlängenbereich eine zentrale Wellenlänge von 1682 nm hat.

19. Medium nach Anspruch 17, welches weiterhin durch einen korrelierenden Druck **gekennzeichnet** ist.

20. Medium nach Anspruch 17, welches weiterhin **dadurch gekennzeichnet ist, dass** ein Gas-Öl-Verhältnis für die Probe basierend auf dem Methan in Gewichtsprozent bestimmt wird.

21. Medium nach Anspruch 17, welches weiterhin **dadurch gekennzeichnet ist, dass** die Probenentgasung basierend auf einer Gewichtsprozentänderung des Methans überwacht wird.

22. Medium nach Anspruch 17, welches weiterhin **dadurch gekennzeichnet ist, dass** basierend auf synthetischen Mischungen von Methan und entgasten Rohölen korreliert wird.

23. Medium nach Anspruch 17, welches weiterhin **dadurch gekennzeichnet ist, dass** eine optische Absorptionsgradmessung mit einem 11nm-Vollbreiten-Halbmaximumsfilter gefiltert wird.

24. Medium nach Anspruch 17, welches weiterhin **dadurch gekennzeichnet ist, dass** der erste Wellenlängenbereich eine zentrale Wellenlänge von 1670 nm und der zweite Wellenlängenbereich eine zentrale Wellenlänge von 1682 nm hat, dass das Methan in Gewichtsprozent, der Druck und die Temperatur mit dem optischen Absorptionsgrad bei dem ersten und zweiten Wellenlängenbereich korreliert werden und dass ein Gas-Öl-Verhältnis basierend auf dem Methan in Gewichtsprozent bestimmt wird.


**Revendications**

1. Procédé pour quantifier en temps réel le pourcentage en poids de méthane dans un environnement d'un puits de forage, comprenant l'obtention du fluide en fond de trou ;
la mesure d'une première valeur d'absorbance optique pour le fluide à une première région de longueurs d'onde associée à un pic du méthane, et **caractérisée par** :

   la mesure d'une seconde valeur d'absorbance optique pour le fluide à une seconde région de longueurs d'onde associée au pic du méthane ; et
   la détermination du pourcentage en poids de méthane pour l'échantillon de fluide d'après les première et seconde valeurs d'absorbance optique mesurées en utilisant une équation de corrélation qui donne le pourcentage en poids de méthane en utilisant les première et seconde valeurs d'absorbance optique, et une valeur de température pour le fluide.

2. Procédé selon la revendication 1, **caractérisé en outre en ce que** la première région de longueurs d'onde a une longueur d'onde centrale de 1670 nanomètres ; et la seconde longueur d'onde a une longueur d'onde centrale de 1682 nanomètres.

**3.** Procédé selon la revendication 1, **caractérisé en outre par** :

la corrélation d'une pression.

**4.** Procédé selon la revendication 1, **caractérisé en outre par** :

la détermination d'un rapport gaz-pétrole pour l'échantillon sur la base du pourcentage en poids de méthane.

**5.** Procédé selon la revendication 1, **caractérisé en outre par** :

le contrôle du nettoyage de l'échantillon sur la base d'un changement du pourcentage en poids de méthane.

**6.** Procédé selon la revendication 1, **caractérisé en outre par** :

une corrélation basée sur des mélanges synthétiques de méthane et de pétroles bruts morts.

**7.** Procédé selon la revendication 1, **caractérisé en outre par** :

le filtrage d'une mesure d'absorbance optique avec un filtre à demi-maximum de pleine largeur de 11 nm.

**8.** Procédé selon la revendication 1, **caractérisé en outre en ce que** la première région de longueurs d'onde a une longueur d'onde centrale de 1670 nanomètres et la seconde longueur d'onde a une longueur d'onde centrale de 1682 nanomètres ;
la corrélation du pourcentage en poids de méthane, de la pression et de la température avec l'absorbance optique aux première et seconde régions de longueurs d'onde ; et
la détermination d'un rapport de gaz et de pétrole sur la base du pourcentage en poids de méthane.

**9.** Appareil pour quantifier en temps réel le pourcentage en poids de méthane dans un environnement de puits de forage, comportant :

un outil (10) pour l'obtention d'un fluide de fond de trou ;
un spectromètre (105) destiné à mesurer une première valeur d'absorbance optique pour le fluide à une première région de longueurs d'onde associée à un pic du méthane et pour mesurer une seconde valeur d'absorbance optique pour le fluide à une seconde région de longueurs d'onde associée au pic du méthane ; et **caractérisé par** :

une fonction de processeur conçue pour déterminer le pourcentage en poids de méthane pour l'échantillon de fluide d'après les première et seconde valeurs d'absorbance optique mesurées en utilisant une équation de corrélation qui donne le pourcentage en poids de méthane en utilisant les première et seconde valeurs d'absorbance mesurées, et une valeur de température pour le fluide.

**10.** Appareil selon la revendication 9, **caractérisé en outre en ce que** la première région de longueur d'onde a une longueur d'onde centrale de 1670 nanomètres ; et la seconde longueur d'onde a une longueur d'onde centrale de 1682 nanomètres.

**11.** Appareil selon la revendication 9, dans lequel la fonction de processeur est en outre **caractérisée par** une fonction pour une corrélation de pression.

**12.** Appareil selon la revendication 9, **caractérisé en outre par** :

une fonction de processeur pour déterminer un rapport de gaz et de pétrole pour l'échantillon sur la base du pourcentage en poids de méthane.

**13.** Appareil selon la revendication 9, **caractérisé en outre par** :

une fonction de processeur pour contrôler le nettoyage d'un échantillon sur la base d'une variation du pourcentage en poids de méthane.

**14.** Appareil selon la revendication 9, dans lequel la fonction de processeur est en outre **caractérisée par** une fonction pour une corrélation basée sur des mélanges synthétiques de méthane et de pétroles bruts morts.

**15.** Appareil selon la revendication 9, **caractérisé en outre par** :

un filtre pour le filtrage d'une mesure d'absorbance optique avec un filtre à demi-maximum d'une pleine largeur de 11 nm.

**16.** Appareil selon la revendication 9, **caractérisé en outre en ce que** la première région de longueur d'onde a une longueur centrale de 1670 nanomètres ; et la seconde longueur d'onde a une longueur d'onde centrale de 1682 nanomètres, la fonction de processeur étant en outre **caractérisée par**

une fonction pour la corrélation d'un pourcentage en poids de méthane, d'une pression et d'une température avec l'absorbance optique aux première et seconde régions de longueur d'onde et une fonction pour déterminer un rapport de gaz et de pétrole sur la base du pourcentage en poids de méthane.

**17.** Support lisible par ordinateur contenant des instructions exécutables qui, lorsqu'elles sont exécutées par un ordinateur, mettent en oeuvre un procédé pour quantifier en temps réel le pourcentage en poids de méthane dans un environnement de puits de forage, comprenant :

l'obtention d'un fluide de fond de trou ;
la mesure d'une première valeur d'absorbance optique pour le fluide à une première région de longueur d'onde associée à un pic du méthane ; et **caractérisé par** :

la mesure d'une seconde valeur d'absorbance optique pour le fluide à une seconde région de longueur d'onde associée au pic du méthane ; et
la détermination du pourcentage en poids de méthane pour l'échantillon de fluide d'après les première et seconde valeurs d'absorbance optique mesurées en utilisant une équation de corrélation qui donne le pourcentage de méthane en utilisant les première et seconde valeurs d'absorbance optique, et une valeur de température pour le fluide.

**18.** Support selon la revendication 17, **caractérisé en outre en ce que** la première région de longueur d'onde a une longueur d'onde centrale de 1670 nanomètres ; et la seconde longueur d'onde a une longueur d'onde centrale de 1682 nanomètres.

**19.** Support selon la revendication 17, **caractérisé en outre par** :

la corrélation d'une pression.

**20.** Support selon la revendication 17, **caractérisé en outre par** :

la détermination d'un rapport gaz-pétrole pour l'échantillon sur la base du pourcentage en poids de méthane.

**21.** Support selon la revendication 17, **caractérisé en outre par** :

le contrôle du nettoyage d'un échantillon sur la base d'une variation du pourcentage en poids de méthane.

**22.** Support selon la revendication 17, **caractérisé en outre par** :

une corrélation basée sur des mélanges synthétiques de méthane et de pétroles bruts morts.

**23.** Support selon la revendication 17, **caractérisé en outre par** :

le filtrage d'une mesure d'absorbance optique avec un filtre à demi-maximum d'une pleine largeur de 11 nm.

**24.** Support selon la revendication 17, **caractérisé en outre en ce que** la première région de longueurs d'onde a une longueur centrale de 1670 nanomètres et la seconde longueur d'onde a une longueur d'onde centrale de 1682 nanomètres ; la corrélation du pourcentage en poids de méthane, d'une pression et d'une température avec l'absorbance optique aux première et seconde régions de longueurs d'onde ; et la détermination d'un rapport gaz-pétrole sur la base du pourcentage en poids de méthane.

FIG. 1

FIG. 2

FIG. 3

**FIG. 4**

FIG. 5

## Equations Correlating Weight Fraction Methane in Mixtures of Crude Oil and Methane to Optical Absorbance and Temperature

**FIG. 6**

Methane Weight Fraction = METHWTF = B0 + B1 * Var1 + B2 * Var2 + B3 * Var3 + B4 * Var4 ...

Regression Summary for Dependent Variable: METHWTF
R= .98093203 $R^2$= .96222765 Adjusted $R^2$= .96151158
F(4,211)=1343.6 p<0.0000 Std.Error of estimate: .04992

|  |  | B |  |
|---|---|---|---|
|  |  | 0.06514 | = B0 = Intercept |
| Var1 = | SQ70_82 | 11.1756 | = B1 |
| Var2 = | TEMP_C | 0.00087 | = B2 |
| Var3 = | SRSA1682 | -2.66167 | = B3 |
| Var4 = | SRSA1670 | 2.63245 | = B4 |

Regression Summary for Dependent Variable: METHWTF
R= .98190316 $R^2$= .96413381 Adjusted $R^2$= .96327986
F(5,210)=1129.0 p<0.0000 Std.Error of estimate: .04876

|  |  | B |  |
|---|---|---|---|
|  |  | 0.03143 | = B0 = Intercept |
| Var1 = | SRSA1670 | 2.53111 | = B1 |
| Var2 = | SRSA1682 | -2.55766 | = B2 |
| Var3 = | SQ70_82 | 11.9135 | = B3 |
| Var4 = | TEMP_C | 0.0019 | = B4 |
| Var5 = | TEMP_SQR | -6.2E-06 | = B5 |

SQ70-82 = SQUARE(Absorbance_at_1670_nm - Absorbance_at_1682_nm)
SRSA1670 = SQRT(Absorbance_at_1670_nm)
SRSA1682 = SQRT(Absorbance_at_1682_nm)
TEMP_C = Temperature in Degrees Centigrade
TEMP_SQR = Square of Temperature in Degrees C

**Equation for Density of Methane [g/cc] as a Function of Pressure and Temperature from 100 - 30,000 psia and 75 - 200 C is fitted by**  Adj. $R^2$= .99911359

|  | B |  |
|---|---|---|
|  | 2.771E-03 | = Intercept |
| P | 2.480E-05 |  |
| $P^2$ | -1.120E-09 | for Pressure in psi |
| $P^3$ | 1.808E-14 |  |
| $T^2$ | -1.308E-07 | for Temperature in C |
| (P/T) | 1.455E-03 |  |
| $(P/T)^2$ | -4.922E-06 |  |
| $(P/T)^3$ | 5.934E-09 |  |

**Equation for Optical Absorbance per mm of Methane as Function of Density and Wavelength at 11 nm FWHM, Center λ range of 1668-1684 nm, for 100-30,000 psia, and 75 - 200 C, is fitted by**  Adj. $R^2$= .94145159

|  | B |  |
|---|---|---|
|  | -19.9061 | = Intercept |
| Methane Density | 0.7747 | for Density in g/cc |
| WaveNumber/1000 | 3.3326 |  |

where, WaveNumber = 10 000 000 / λ (nm)

Equations Relating Gas Oil Ratio, GOR, to Weight Fraction of Methane, $f_M$, and Stock Tank Density, $p_O$, of Oil
1 bbl = 0.159 $m^3$=5.615 cu ft=42 U.S. gal
1 Standard Cubic Foot (SCF) of Methane Gas at 14.7 psia & 60°F is 0.042358 lbs = 19.21327 grams.
Density of Methane at 60 F and 14.7 psia is 0.0006787 gr/cc = 0.042358 lbm/$ft^3$.
Letting V = Volume, W = Weight, p = Density, and using subscripts M for Methane and O for Oil,
GOR = $V_{Methane}$ [SCF] / $V_{Oil}$ [bbls] = { $W_M$ / (19.21 g/SCF) } / {( $W_O$/ $p_O$ ) ( 1 bbl / 158 983 cc )}
Letting $f_M$ = Weight Fraction of Methane,
GOR = 8274.62 $p_O$ / ( 1 / $f_M$ - 1 )
$f_M$ = $W_M$ / ( $W_M$ + $W_O$ ) = $p_M V_M$ / ( $p_M V_M$ + $p_O V_O$ )  so  $W_O$ = $W_M$ / ( 1 / $f_M$ - 1 ) which substitutes into above.
$f_M$ = 1 / ( 1 + 8274.62 * $p_O$ / GOR ) where $W_G$ and $W_O$ are in grams, $p_O$ is in g/cc, and $f_M$ = Wt. Frac. of Methane

**Figure 7**

| Prepare training set of high-resolution absorption spectra of synthetic mixtures of methane and dead crude oils. | ← — **810** |

↓

| Degrade high-resolution spectra to 11 nm FWHM corresponding to best available high-temperature filters. | ← — **812** |

↓

| Determine best correlating center wavelengths (1670 nm and 1682 nm) to weight fraction of methane in crude oils. | ← — **814** |

↓

| Determine correlation equations that use best correlating wavelengths and/or temperature and/or pressure. | ← — **816** |

↓

| Obtain a first absorbance of a downhole fluid at a first wavelength region (1670 nm) associated with a methane peak. | ← — **818** |

↓

| Obtain a 2nd absorbance of a downhole fluid at a 2nd wavelength region (1682 nm) associated with a methane peak. | ← — **820** |

↓

| Using derived correlation equation, determine weight fraction of methane and GOR for the downhole fluid. | ← — **822** |

**Figure 8**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4994671 A, Safinya
- US 5892586 A, Thony
- US 5939717 A, Mullins
- US 6476384 B

**Non-patent literature cited in the description**

- **DONG C. et al.** In-Situ Contamination Monitoring and GOR Measurement of Formation Fluid Samples. *PROCEEDINGS SPE ASIA PACIFIC OIL AND GAS CONFERENCE AND EXHIBITION,* 08 October 2002, 635-643